# EUROPÄISCHE PATENTSCHRIFT

(11) **EP 0 784 645 B1**
(45) Veröffentlichungstag und Bekanntmachung des Hinweises auf die Patenterteilung: **12.05.1999**
(21) Anmeldenummer: 95935382.2
(22) Anmeldetag: 26.09.1995
(51) Int. Cl.: C08G 73/02, C08G 73/04

(54) **VERFAHREN ZUR HERSTELLUNG VON OLIGOAMINEN ODER POLYAMINEN**
PROCESS FOR PRODUCING OLIGOAMINES OR POLYAMINES
PROCEDE DE PREPARATION D'OLIGOAMINES OU DE POLYAMINES

(30) Priorität: 06.10.1994 DE 4435688
(43) Veröffentlichungstag der Anmeldung: 23.07.1997
(73) Patentinhaber: BASF AKTIENGESELLSCHAFT, 67056 Ludwigshafen (DE)
(72) Erfinder: MOHR, Jürgen, D-67269 Grünstadt (DE); KNAUF, Wolfgang, D-67117 Limburgerhof (DE); BALZER, Wolf-Dieter, D-67069 Ludwigshafen (DE); OPPENLÄNDER, Knut, D-67061 Ludwigshafen (DE); SLOTMAN, Wilhelmus, D-67063 Ludwigshafen (DE)
(86) Internationale Anmeldenummer: EP9503809
(87) Internationale Veröffentlichungsnummer: WO9611225

(56) Entgegenhaltungen:
- EP-A- 0 147 743
- FR-A- 2 187 884
- FR-A- 2 335 492

## Beschreibung

Die vorliegende Erfindung betrifft ein verbessertes Verfahren zur Herstellung von bestimmten Oxalkylaten von Oligoaminen oder Polyaminen durch zweistufige Arbeitsweise, wobei in der ersten Stufe die Anlagerung eines Moleküls Alkylenoxid pro NH-Gruppierung an die Oligoamine bzw. Polyamine in Gegenwart von Wasser, Alkoholen oder Säuren oder einer Mischung hieraus in Abwesenheit eines neutralen oder basischen Katalysators erfolgt und wobei in der zweiten Stufe nach der Entfernung von Wasser und Säuren in Gegenwart eines üblichen neutralen oder basischen Katalysators mit weiterem Alkylenoxid umgesetzt wird.

Aus der DE-B 22 27 546 ist ein zweistufiges Verfahren zur Herstellung von oxalkylierten Polyalkyleniminen bekannt, bei dem man in der ersten Stufe auf ein Polyalkylenpolyamin in Gegenwart von 1 bis 50 Gew.-% Wasser so viel Alkylenoxid einwirken läßt, daß unter Absättigung sämtlicher Wasserstoffatome bindender Valenzen der Stickstoffatome der entsprechende Aminoalkohol entsteht, und bei dem in der zweiten Stufe nach Entfernung des Wassers ein alkalischer Katalysator zugefügt wird, woran sich die weitere Oxalkylierung anschließt.

Die Weiterreaktion des Aminoalkohols in der zweiten Stufe muß bekanntlich in Abwesenheit von Wasser durchgeführt werden, da sich sonst übermäßig viele unerwünschte Nebenprodukte mit Glykol- und Polyglykolstruktur bilden. Die weitere Oxalkylierung am reinen polymeren Aminoalkohol, also beispielsweise in der Schmelze, läuft jedoch nur noch sehr langsam ab. Insbesondere kommt es zu Beginn dieser Weiterreaktion zu hochviskosen Zuständen, die schwer zu kontrollieren und zu überwinden sind, dies führt zu stark schwankenden Reaktionszeiten und zu Produkten, die sich in ihrer Viskosität stark unterscheiden.

Es bestand daher die Aufgabe, Reaktionsbedingungen zu finden, unter denen sich die polymeren Aminoalkohole aus der ersten Stufe des beschriebenen Herstellungsverfahrens auf wirtschaftliche und effiziente Art und Weise mit kurzen Reaktionszeiten zu reproduzierbaren Produkten oxalkylieren lassen.

Demgemäß wurde ein Verfahren zur Herstellung von Oxalkylaten von Oligoaminen oder Polyaminen aus der Gruppe bestehend aus Polyalkylenpolyaminen mit 3 bis 10 N-Atomen pro Molekül, Polyvinylaminen mit einem gewichtsgemittelten Molekulargewicht von 600 bis 10 000 000 und Polyethyleniminen mit einem gewichtsgemittelten Molekulargewicht von 2000 bis 50 000 durch zweistufige Arbeitsweise, wobei in der ersten Stufe die Anlagerung eines Moleküls Alkylenoxid pro NH-Gruppierung an die Oligoamine bzw. Polyamine in Gegenwart von Wasser, Alkoholen oder Säuren oder einer Mischung hieraus in Abwesenheit eines neutralen oder basischen Katalysators erfolgt und wobei in der zweiten Stufe nach der Entfernung von Wasser und Säuren in Gegenwart eines üblichen neutralen oder basischen Katalysators mit weiterem Alkylenoxid umgesetzt wird, gefunden, welches dadurch gekennzeichnet ist, daß man vor der Durchführung der zweiten Stufe ein organisches Lösungs- oder Verdünnungsmittel oder eine Mischung solcher Mittel aus der Gruppe
(f) aromatische Kohlenwasserstoffe
zusetzt.

Das genannte Lösungs- oder Verdünnungsmittel wird vorzugsweise in einer Menge von 2 bis 400 Gew.-Teilen, insbesondere 5 bis 200 Gew.-Teilen, vor allem 10 bis 100 Gew.-Teilen, bezogen auf das Gewicht des Anlagerungsproduktes aus der ersten Stufe, zugegeben.

Als aromatische Kohlenwasserstoffe (f) kommen insbesondere Benzol, Toluol, Xylol, Mesitylen, Styrol, Indan, Inden sowie technische Aromatenschnitte, die überwiegend oder ausschließlich aus derartigen aromatischen Kohlenwasserstoffen bestehen, z.B. Naphtha (eine Mischung aus Alkylbenzolen) und "Solvent Naphtha schwer", in Betracht. Von besonderem Interesse ist Xylol, wobei hierunter sowohl die isomerenreinen Verbindungen o-, m- und p-Xylol als auch das technische Gemisch hieraus zu verstehen sind.

Als nach dem erfindungsgemäßen Verfahren zu oxalkylierende Oligo- oder Polyamine kommen Polyalkylenpolyamine mit 3 bis 10, vorzugsweise 3 bis 7 N-Atomen pro Molekül wie Diethylentriamin, Triethylentetramin oder Tetraethylenpentamin, weiterhin Polyvinylamine mit einem gewichtsgemittelten Molekulargewicht von 600 bis 10 000 000, vorzugsweise 2000 bis 7 000 000, sowie weiterhin Polyethylenimine mit einem gewichtsgemittelten Molekulargewicht von 2000 bis 50000, vorzugsweise 5000 bis 25000, in Betracht.

Bei der erfindungsgemäßen Oxalkylierung kommen sämtliche üblichen 1,2-Alkylenoxide, insbesondere Ethylenoxid, Propylenoxid und Butylenoxid, wobei sowohl 1,2- als auch 2,3-Butylenoxid verwendet werden kann, daneben aber auch Styroloxid und Cyclohexenoxid sowie eine Mischung aus den genannten Alkylenoxiden in Betracht.

Das erfindungsgemäße zweistufige Oxalkylierungsverfahren gemäß Oberbegriff wird wie üblich durchgeführt. Dazu wird in der ersten Stufe ein Molekül Alkylenoxid pro NH-Gruppierung in stöchiometrischer Umsetzung angelagert, wobei man vorzugsweise in Wasser, welches die Ausgangsamine normalerweise am besten löst, oder in einem wäßrigen Medium und üblicherweise bei erhöhten Temperaturen, etwa bei 50 bis 130°C, insbesondere 70 bis 120°C, und unter Druck, etwa bei 1,1 bis 10 bar, insbesondere 2 bis 5 bar, beispielsweise in einem Autoklaven, arbeitet. Als Alkohole und/oder Säuren, die im Reaktionsmedium alleine oder vorzugsweise zusammen mit Wasser als Lösungsmittel oder Reaktionsbeschleuniger mit anwesend sein können, kommen beispielsweise C₁- bis C₄-Alkanole wie die oben unter (a) genannten bzw. übliche Mineralsäuren, z.B. Salzsäure oder Schwefelsäure, oder übliche Carbonsäuren, z.B. Essigsäure, in Betracht.

In der zweiten Stufe wird nach der Entfernung von Wasser und Säuren und gegebenenfalls von Alkoholen, wenn solche hier als Lösungs oder Verdünnungsmittel nicht gewünscht sind, ein üblicher neutraler oder basischer Katalysator zugegeben. Als basische Katalysatoren, welche bevorzugt werden, eignen sich insbesondere Alkalimetallhydroxide wie NaOH und KOH und Alkalimetallalkoholate wie Natrium- oder Kaliummethanolat, -ethanolat, -isopropylat und -tert.-butylat. Als neutrale Katalysatoren sind beispielsweise Schichtverbindungen wie gegebenenfalls modifizierter Hydrotalcit zu nennen. Man arbeitet in der zweiten Stufe in der Regel bei erhöhten Temperaturen von etwa 70 bis 180°C, insbesondere 90 bis 150°C, und ähnlichen Drücken wie in der ersten Stufe, beispielsweise auch in einem Autoklaven.

Die Entfernung von Wasser und auch Säuren und gegebenenfalls Alkoholen vor Durchführung der zweiten Stufe wird entweder durch azeotrope Abdestillation mittels des erfindungsgemäß zugesetzten Lösungs- oder Verdünnungsmittels, oder durch Abdestillieren im Vakuum vor Zugabe des Lösungs- oder Verdünnungsmittels oder durch Abdestillation im Vakuum nach Zugabe des Lösungs- oder Verdünnungsmitteln vorgenommen, wobei letzteres vor, während oder nach Durchführung der ersten Stufe zugegeben werden kann und wobei letzteres einen ausreichend hohen Siedepunkt aufweisen muß.

Nach dem erfindungsgemäßen Verfahren können Oxalkylate von Oligo- oder Polyaminen hergestellt werden, die eine beliebige Anzahl von Alkylenoxid-Einheiten pro ursprünglicher NH-Gruppierung aufweisen. Vorzugsweise kommen hier 3 bis 300, insbesondere 4 bis 200, vor allem 10 bis
100 Alkylenoxid-Einheiten pro ursprünglicher NH-Gruppierung in Betracht.

In Anwesenheit der erfindungsgemäß zugesetzten Lösungs- oder Verdünnungsmittel läßt sich die zweite Stufe der Oxalkylierung wesentlich besser kontrollieren und steuern, da die sonst auftretenden Viskositätsprobleme vermieden werden. Vor allem treten wesentlich kürzere Reaktionszeiten auf.

Bei azeotroper Abdestillation des Wassers vor Durchführung der zweiten Stufe gelingt die Wasserentfernung im Gegensatz zur Wasserentfernung beispielsweise aus der Schmelze wesentlich schneller und ist nahezu vollständig. Besonders überraschend war, daß die relativ unpolaren und aprotischen Lösungsmittel aus der Gruppe (f) derart geeignet sind, die notwendige Entwässerung von hochpolaren Verbindungen, nämlich der entsprechenden Aminoalkohle aus der ersten Stufe der Oxalkylierung, zu bewirken.

Die nach dem erfindungsgemäßen Verfahren erhaltenen Produkte können insbesondere als Spalter für Rohölemulsionen verwendet werden. Dabei spalten die Produkte, welche die verwendeten Lösungs- oder Verdünnungsmittel noch enthalten, Rohölemulsionen in vielen Fällen sogar besser als analoge Produkte, die ohne die beschriebenen Lösungs- oder Verdünnungsmittel hergestellt und denen solche Mittel nachträglich zugesetzt wurden. Ein derartiger "synergistischer Effekt" konnte durch Abmischung von kommerziell verfügbaren analogen oxalkylierten Oligo- oder Polyaminen, welche ohne die beschriebenen Lösungs- oder Verdünnungsmittel hergestellt wurden, mit den besagten Lösungs- oder Verdünnungsmitteln nachgewiesen werden. Daher ist ein Verfahren zur Herstellung von Spaltern für Rohölemulsionen, welches dadurch gekennzeichnet ist, daß man Oxalkylate von Oligoaminen oder Polyaminen aus der Gruppe bestehend aus Polyalkylenpolyaminen mit 3 bis 10 N-Atomen pro Molekül, Polyvinylaminen mit einem gewichtsgemittelten Molekulargewicht von 600 bis 10.000.000 und Polyethyleniminen mit einem gewichtsgemittelten Molekulargewicht von 2000 bis 50.000 durch zweistufige Arbeitsweise gemäß Anspruch 1 oder 2 unter Verwendung von organischen Lösungs- oder Verdünnungsmittel aus der Gruppe der Verbindungen (f) gemäß Anspruch 1 herstellt und die eingesetzten Lösungs- oder Verdünnungsmittel in den erhaltenen Produkten für die Spaltung von Rohölemulsionen beläßt, ebenfalls Gegenstand der vorliegenden Anmeldung.

### Beispiel 1a

### Monoalkoxylierung eines Polyethylenimins in Wasser (Erste Stufe) und azeotrope Abdestillation des Wassers nach Zugabe von Xylol

In einem Autoklaven wurden 43 g eines Polyethylenimins mit einem gewichtsgemittelten Molekulargewicht von ca. 20 000 zusammen mit 43 g Wasser vorgelegt. Unter Rühren wurden dann bei einer Temperatur von 90-100°C und einem Druck von max. 4 bar 58 g Propylenoxid während 30 min zugegeben. Man rührte noch 1 h bei dieser Temperatur nach, kühlte auf ca. 80°C ab, fügte 20 g 50 gew.-%ige wäßrige KOH-Lösung und 100 g Xylol zu und trennte sämtliches Wasser durch azeotrope Destillation ab.

### Beispiel 1b

### Weitere Oxalkylierung (Zweite Stufe) in Gegenwart von Xylol

In einem Autoklaven wurde die Vorstufe aus Beispiel la bei einer Temperatur von 130-140°C und einem Druck bis ca. 4,5 bar mit ca. 2610 g Propylenoxid umgesetzt. Die Reaktionszeit betrug hierbei 1230 min.

### Vergleichsbeispiel A

Beispiel 1a und 1b wurden ohne den Zusatz von Xylol vor der Durchführung der zweiten Stufe wiederholt. Das Wasser wurde hierbei im Vakuum (bis ca. 10 mbar) bei 120°C abdestilliert. Die Reaktionszeit in der zweiten Stufe betrug 3600 min.

## Patentansprüche

1. Verfahren zur Herstellung von Oxalkylaten von Oligoaminen oder Polyaminen aus der Gruppe bestehend aus Polyalkylenpolyaminen mit 3 bis 10 N-Atomen pro Molekül, Polyvinylaminen mit einem gewichtsgemittelten Molekulargewicht von 600 bis 10 000 000 und Polyethyleniminen mit einem gewichtsgemittelten Molekulargewicht von 2000 bis 50 000 durch zweistufige Arbeitsweise, wobei in der ersten Stufe die Anlagerung eines Moleküls Alkylenoxid pro NH-Gruppierung an die Oligoamine bzw. Polyamine in Gegenwart von Wasser, Alkoholen oder Säuren oder einer Mischung hieraus in Abwesenheit eines neutralen oder basischen Katalysators erfolgt und wobei in der zweiten Stufe nach der Entfernung von Wasser und Säuren in Gegenwart eines üblichen neutralen oder basischen Katalysators mit weiterem Alkylenoxid umgesetzt wird, dadurch gekennzeichnet, daß man vor der Durchführung der zweiten Stufe ein organisches Lösungs- oder Verdünnungsmittel oder eine Mischung solcher Mittel aus der Gruppe
(f) aromatische Kohlenwasserstoffe
zusetzt.

2. Verfahren nach Anspruch 1, dadurch gekennzeichnet, daß man das organische Lösungs- oder Verdünnungsmittel in einer Menge von 2 bis 400 Gew.-Teilen, bezogen auf das Gewicht des Anlagerungsproduktes aus der ersten Stufe, zugibt.

3. Verfahren nach Anspruch 1 oder 2, dadurch gekennzeichnet, daß man als organisches Lösungs- oder Verdünnungsmittel Xylol einsetzt.

4. Verfahren nach den Ansprüchen 1 bis 3, wobei als Alkylenoxid Ethylenoxid, Propylenoxid, Butylenoxid oder eine Mischung hieraus eingesetzt wird.

5. Verfahren zur Herstellung von Spaltern für Rohölemulsionen, dadurch gekennzeichnet, daß man Oxalkylate von Oligoaminen oder Polyaminen aus der Gruppe bestehend aus Polyalkylenpolyaminen mit 3 bis 10 N-Atomen pro Molekül, Polyvinylaminen mit einem gewichtsgemittelten Molekulargewicht von 600 bis 10.000.000 und Polyethyleniminen mit einem gewichtsgemittelten Molekulargewicht von 2000 bis 50.000 durch zweistufige Arbeitsweise gemäß Anspruch 1 oder 2 unter Verwendung von organischen Lösungs- oder Verdünnungsmittel aus der Gruppe der Verbindungen (f) gemäß Anspruch 1 herstellt und die eingesetzten Lösungs- oder Verdünnungsmittel in den erhaltenen Produkten für die Spaltung von Rohölemulsionen beläßt.

## Claims

1. A process for preparing alkoxylates of oligoamines or polyamines from the group consisting of polyalkylenepolyamines with 3 to 10 nitrogen atoms per molecule, polyvinylamines with a weight average molecular weight of from 600 to 10,000,000 and polyethyleneimines with a weight average molecular weight of from 2000 to 50,000 by a two-stage procedure where, in the first stage, one molecule of alkylene oxide per NH group is added onto the oligoamines or polyamines in the presence of water, alcohols or acids or a mixture thereof in the absence of a neutral or basic catalyst and, in the second stage, after removal of water and acids, reaction with further alkylene oxide is carried out in the presence of a conventional neutral or basic catalyst, wherein, before the second stage is carried out, an organic solvent or diluent or a mixture thereof from the group of
(f) aromatic hydrocarbons,
is added.

2. A process as claimed in claim 1, wherein the organic solvent or diluent is added in an amount of from 2 to 400 parts by weight, based on the weight of the adduct from the first stage.

3. A process as claimed in claim 1 or 2, wherein xylene is employed as organic solvent or diluent.

4. A process as claimed in any of claims 1 to 3, where ethylene oxide, propylene oxide, butylene oxide or a mixture thereof is employed as alkylene oxide.

5. A process for preparing agents for breaking crude oil emulsions, which comprises preparing alkoxylates of oligoamines or polyamines from the group consisting of polyalkylenepolyamines with 3 to 10 nitrogen atoms per molecule, polyvinylamines with a weight average molecular weight of from 600 to 10,000,000 and polyethyleneimines with a weight average molecular weight of from 2000 to 50,000 by a two-stage procedure as claimed in claim 1 or 2 using organic solvents or diluents from the group of compounds (f) as set forth in claim 1, and leaving the solvents or diluents employed in the resulting products for breaking crude oil emulsions.

## Revendications

1. Procédé pour la préparation d'oxalkylates d'oligoamines ou de polyamines choisies dans l'ensemble constitué par les polyalkylènepolyamines ayant de 3 à 10 atomes d'azote par molécule, les polyvinylamines ayant une masse moléculaire moyenne en poids de 600 à 10 000 000 et les polyéthylène-imines ayant une masse moléculaire moyenne en poids de 2 000 à 50 000, par un mode opératoire en deux étapes, dans la première étape étant effectuée la fixation par addition d'une molecule d'oxyde d'alkylène, par groupement NH, sur les oligoamines ou polyamines en présence d'eau, d'alcools ou d'acides ou d'un mélange de ceux-ci, en absence d'un catalyseur neutre ou basique, et dans la seconde étape étant effectuée une réaction avec une nouvelle quantité d'oxyde d'alkylène, après l'élimination d'eau et d'acides, en présence d'un catalyseur neutre ou basique usuel, caractérisé en ce que, avant d'effectuer la seconde étape on ajoute un solvant ou diluant organique choisi dans le groupe
(f) des hydrocarbures aromatiques
ou un mélange de telles substances.

2. Procédé selon la revendication 1, caractérisé en ce que l'on ajoute le solvant ou diluant organique en une quantité de 2 à 400 parties en poids, par rapport au poids du produit d'addition provenant de la première étape.

3. Procédé selon la revendication 1 ou 2, caractérisé en ce que l'on utilise comme solvant ou diluant organique le xylène.

4. Procédé selon les revendications 1 à 3, dans lequel on utilise en tant qu'oxyde d'alkylène l'oxyde d'éthylène, l'oxyde de propylène, l'oxyde de butylène ou un mélange de ceux-ci.

5. Procédé pour la préparation de désémulsifiants pour émulsions de pétrole brut, caractérisé en ce que l'on prépare selon la revendication 1 des oxalkylates d'oligoamines ou de polyamines choisies dans l'ensemble constitué par les polyalkylènepolyamines ayant de 3 à 10 atomes d'azote par molécule, les polyvinylamines ayant une masse moléculaire moyenne en poids de 600 à 10 000 000 et les polyéthylène-imines ayant une masse moléculaire moyenne en poids de 2 000 à 50 000, par un mode opératoire en deux étapes selon la revendication 1 ou 2 en utilisant des solvants ou diluants organiques choisis dans le groupe des composés (f), et on laisse dans les produits obtenus les solvants ou diluants utilisés, pour la désémulsification d'émulsions de pétrole brut.
